(19) Europäisches Patentamt
European Patent Office
Office européen des brevets

(11) Publication number: **0 547 484 A1**

(12) **EUROPEAN PATENT APPLICATION**

(21) Application number: **92120936.7**

(22) Date of filing: **09.12.92**

(51) Int. Cl.5: **C12P 21/04**, C07K 11/02, //(C12P21/04,C12R1:645)

(30) Priority: **19.12.91 JP 353977/91**

(43) Date of publication of application: **23.06.93 Bulletin 93/25**

(84) Designated Contracting States: **DE FR GB IT**

(71) Applicant: **Nippon Kayaku Kabushiki Kaisha**
**11-2, Fujimi-cho 1 chome Chiyoda-ku**
**Tokyo(JP)**

(72) Inventor: **Moteki, Yoshi**
**239, Iwahanamati, Takasaki-shi**
**Gunma-ken(JP)**

(74) Representative: **Türk, Gille, Hrabal, Leifert**
**Brucknerstrasse 20**
**W-4000 Düsseldorf 13 (DE)**

(54) **Process for producing antibiotic R106-I.**

(57) [ Object ] To efficiently obtain a pulified antibiotic R106-I which is useful as a remedy for fungal infections.

[ Constitution ] A process for producing an antibiotic R106-I by fermentation which comprises culturing a strain capable of producing the antibiotic R106-I in an isoleucine-medium and collecting the antibiotic R106-I thus produced from the culture. The isoleucine may be either added to the medium at the beginning as one of the components of the medium or continuously or intermittently added thereto during the culture.

EP 0 547 484 A1

Background of the Invention:

[ Field of Industrial Application ]

This invention relates to a process for producing an antibiotic R106-I which is useful as a remedy for fungal infections.

[ Prior Art ]

An example of known processes for producing an antibiotic R106-I represented by the following structural formula:

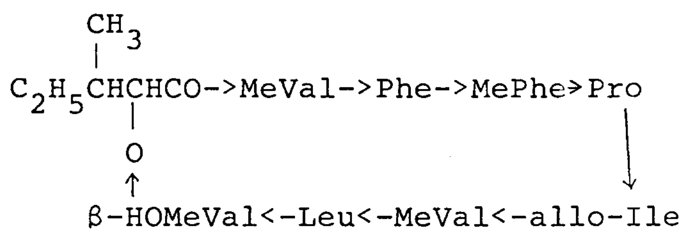

wherein MeVal represents N-methylvaline, Phe represents phenylalanine, MePhe represents N-methylphenylalanine, Pro represents proline, allo-Ile represents allo-isoleucine, Leu represents leucine and β-HOMeVal represents β-hydroxy-N-methylvaline,
and analogs thereof comprises culturing a strain belonging to the genus Aureobasidium, such as Aureobasidium pullulans No. R106 strain [FERM BP-1938] in a medium containing nutritional sources and collecting the antibiotic R106-I thus produced from the culture [refer to U.S.P. No. 5,057,493]. However, this process is unsatisfactory in the collection, purification and production of the antibiotic R106-I.

Summary of the Invention:

[ Problems to be Solved by the Invention ]

The present invention aims at providing a process for producing the antibiotic R106-I at an elevated efficiency.

[ Means for Solving the Problems ]

The present inventors have found out a process for producing the antibiotic R106-I by fermentation which is characterized by culturing a microorganism belonging to the genus Aureobasidium and capable of producing the antibiotic R106-I in a nutrient isoleucine-medium and efficiently collecting the antibiotic R106-I accumulated in the culture, thus completing the present invention.

Detailed Description of the Invention:

As a particular example of the microorganisms to be used in the present invention, Aureobasidium pullulans No. R106 strain [FERM BP-1938] disclosed in the above-mentioned U.S.P. No. 5,057,493 may be cited. Further, variants of this strain are usable. Furthermore, any microorganism belonging to the genus Aureobasidium and capable of producing the antibiotic R106-I can be used in the present invention.

The term "an isoleucine-medium" to be used herein means a medium to which isoleucine is separately added in addition to nitrogen sources employed in a common medium. Namely, the isoleucine contained in common nitrogen sources, for example, yeast extract and meat extract is excluded from the "isoleucine added" in the present invention.

The antibiotic R106-I according to the present invention may be produced by a usual cultivation method except that an isoleucine-medium at the beginning is used or isoleucine is added to a medium during cultivation. The medium to be used herein may be a common one containing carbon sources, nitrogen sources, inorganic matters and other trace nutrients required by the employed strain. Examples of the carbon source include glucose, fructose, sucrose, maltose, lactose, dextrin, starch, starch syrup, molasses,

glycerol, oils and fats and organic acids. Examples of the nitrogen source include organic and inorganic nitrogen compounds including soybean meal, cotton seed meal, corn steep liquor, peptone, casein, casamino acids, yeast extract, embryo, meat extract urea, amino acids except isoleucine, and ammonium salts. As the inorganic substances inorganic salts such as sodium, potassium, calcium and magnesium salts and phosphates may be used either alone or in the form of a mixture. Furthermore, the medium may optionally contain heavy metals such as iron, copper, zinc, manganese and cobalt salts and vitamins such as biotin and vitamin $B_1$, if necessary. Furthermore, surfactants such as silicone oil or polyalkylene glycol may be optionally added to the medium.

For culture conventional techniques employed for producing an antibiotic by culturing a micro-organism may be used. A liquid culture method, in particular, shaken culture or submerged aeration/agitation culture is suitable therefor. The culture temperature may preferably range from 15 to 30 °C in general, while the culture pH may preferably range from 2 to 8 in general. The culture period usually ranges from 1 to 14 days, though it varies depending on the culture conditions. The amount of the isoleucine to be added to the medium varies depending on the antibiotic R106-I-producing strain to be used, and the optimum content thereof should be determined by taking other components of the medium into consideration. In usual, isoleucine may be added in such an amount as to give a concentration of from 0.02 to 2.0 %, preferably from 0.03 to 1.0 % and still preferably from 0.05 to 0.6 % (w/v), based on the total amount of the medium. Regarding the time for adding isoleucine, the medium may contain isoleucine as one of the components thereof at the beginning of the culture of the strain producing the antibiotic R106-I, or alternately isoleucine may be continuously or intermittently added to the medium during the culture. When a carbon source such as glucose, maltose or sucrose or a nitrogen source such as peptone or yeast extract is added to the medium during the culture, isoleucine may be added simultaneously therewith.

According to the culture of the present invention, the antibiotic R106-I accumulated in the culture increases and by-products, of which the separation is difficult, decrease.

After the completion of the culture, the antibiotic R106-I accumulated in the culture is collected therefrom by taking advantage of the physicochemical properties of the antibiotic R106-I. Namely, the antibiotic R106-I, which is contained in mycelial cake and the culture filtrate, can be separated and collected by extraction with a dydrophobic organic solvent such as chloroform, ethyl acetate, butyl acetate, butanol or methyl isobutyl ketone. Alternately, the culture may be separated into mycelial cake and the filtrate by filtering or centrifuging and then the antibiotic R106-I is extracted and collected. The antibiotic R106-I may be separated and collected from the culture filtrate by extracting with the above-mentioned hydrophobic organic solvent. Alternately, the culture filtrate may be adsorbed on a supporting resin such as activated charcoal, cellulose powder or adsorptive resin and then eluted with a suitable solvent. The antibiotic R106-I may be eluted from such a supporting resin with the a water-soluble organic solvent such as aqueous alcohol or water-containing acetone. The antibiotic R106-I may be separated and collected from mycelical cake by extracting with a hydrophilic organic solvent such as acetone or ethanol. Subsequently, the antibiotic R106-I may be further purified by a known method for isolating and purifying fat-soluble antibiotics. For example, column chromatography with supporting resin such as silica gel, activated aluminium or adsorptive resins may be employed therefor. In the column chromatography with silica gel as a supporting resin, the antibiotic R106-I may be eluted by an eluent such as a hydrocarbon (for example hexane and heptane) chloroform, ethyl acetate, acetone, an ($C_1 \sim C_4$) alcohol (for example, methanol, ethanol, isopropanol) or water or a solvent mixture comprising them. A mixture of a hydrocarbon and an alcohol is preferable. High performance liquid chromatography may be advantageously used for the separation and purification. Example of the supporting resin usable therein include silica gel, silica gel with chemical bonds such as octadecyl, amino or octyl groups bonded thereto, and poly-styrenic porous polymer gels. Example of the mobile phase to be used in the elution include hexane, isopropyl alcohol, water-containing methanol and aqueous acetonitrile. Further, the counter-current distribution method, which is a separation and purification method based on distribution between liquid phases, may be advantageously used therefor. As the distribution liquid system, a solvent mixture such as a hexane/ethyle acetate/acetonitrile or chloroform/methanol/water system may be used.

[ Example ]

To further illustrate the present invention in greater detail, the following Examples will be given. It is to be noted, however, that the present invention is not restricted thereto.

Example 1

One platinum loop from a slant culture of the No. R106 strain (FERM BP-1938) was inoculated into 100 ml of a liquid medium Difco Yeast Nitrogen Base: 0.67 % (w/v), glucose: 2.0 % (w/v) contained in a 500-ml Erlenmeyer flask and shaken at 27 °C for 2 days to thereby give a seed culture liquor. Then, 1.0 ml of this seed culture liquor was inoculated into 100 ml of a liquid medium A with 0.1 % of L-isoleucine added in a 500-ml Erlenmeyer flask and culture under shaking on a rotary shaker at 210 rpm at 25 °C for 3 days. Further, 20 ml of an additional liquid medium B was added to the culture liquor, followed by culturing under shaking on a rotary shaker at 210 rpm for additional 4 days.

Note (1)

medium A: glucose: 2 %, ammonium sulfate: 0.5 %, potassium primary phosphate: 0.15 %, magnesium sulfate heptahydrate: 0.05 %, sodium chloride: 0.01 %, calcium chloride: 0.01 %, (each concentration being expressed in w/v), ferric chloride: 0.5 μg/ml, zinc sulfate: 0.5 μg/ml.

Note (2)

medium B: glucose: 10 %, ammonium sulfate: 2.5 %, polypeptone: 5 %, potassium primary phosphate: 0.75 %, magnesium sulfate heptahydrate: 0.25 %, calcium chloride: 0.05 %, sodium chloride: 0.05 % (each concentration being expressed in w/v), ferric chloride: 2.5 μg/ml, zinc sulfate: 2.5 μg/ml.

5 l of the culture liquor thus obtained was collected and separated into the filtrate and mycelial cake by centrifuging. Then the obtained mycelial cake were extracted by adding 1 l of ethanol. The ethanol extract was concentrated under reduced pressure. After distilling off the ethanol, the residue was extracted with 0.3 l portions of ethyl acetate twice. The extract was concentrated to dryness under reduced pressure and the residue was dissolved in chloroform. The chloroform solution was poured into 300 ml of a silica gel column, which had been previously saturated with hexane, washed with 600 ml of hexane and then eluted and developed with 1.2 l of a mixture of hexane with isopropanol (7 : 3). The active fraction thus eluted was concentrated under reduced pressure. The obtained residue was dissolved in 20 ml of acetonitrile and subjected to preparative high performance liquid chromatography [column: Soken Pack $C_{18}$ (mfd. by Soken Kagaku K.K.) 2 cm x 50 cm, mobile phase: 70 % (v/v) aqueous acetonitrile] in 10 portions. Thus an active fraction having a purity of 95 % or above was collected. This fraction was further concentrated under reduced pressure and thus 0.8 g of the antibiotic R106-I was obtained in the form of a white powder.

Example 2

1.0 ml of a seed culture liquor of the No. R106 strain, which had been prepared in the same manner as the one described in the above Example 1, was inoculated into 100 ml of the liquid medium A and cultured under shaking on a rotary shaker at 210 rpm at 25 °C. Similar to Example 1, 20 ml of the additional liquid medium B was added to the culture liquor. Further, 5-ml portions of a 1.0 % L-isoleucine solution was added thereto on the 3rd, 4th and 5th days of the culture. From the 5th day of the culture, it was further cultured under shaking on a rotary shaker at 210 rpm for additional 2 days.

5 l of the culture liquor thus obtained was collected and subjected to the same separation and purification procedures as those carried out in the above Example 1. As a result, 0.9 g of the antibiotic R106-I was obtained in the form of a white powder as an active fraction.

Referential Example 1

1.0 ml of a seed culture liquor of the No. R106 strain, which had been prepared in the same manner as the one described in the above Example 1, was inoculated into 100 ml of the liquid medium A and cultured under shaking on a rotary shaker at 210 rpm at 25 °C for 3 days. 20 ml of the additional liquid medium B was added to the culture liquor, followed by culturing under shaking on a rotary shaker at 210 rpm at 25 °C for additional 4 days. 5 l of the culture liquor thus obtained was collected and subjected to the same separation and purification procedures as those carried out in the above Example 1. As a result, 0.5 g of the antibiotic R106-I was obtained in the form of a white powder as an active fraction.

Table 1 shows the yields of the antibiotic R106-I in the Examples 1 and 2, wherein the medium with L-isoleucine added was employed, and that in the Referential Example 1, wherein the medium without addition of L-isoleucine were employed, as a control.

[ Table 1 ]

Table 1

| | Yield of R106-I (g) (purity: 95 % or above) |
|---|---|
| Example 1 | 0.8 |
| Example 2 | 0.9 |
| Referential Example 1 | 0.5 |

As Table 1 clearly shows, the process of the present invention is superior to the control one.

[ Effects of the Invention ]

According to the present invention, a purified antibiotic R106-I can be obtained with high yields.

## Claims

1. A process for producing an antibiotic R106-I by fermentation which comprises culturing a strain belonging to the genus Aureobasidium and capable of producing the antibiotic R106-I in an isoleucine-medium and collecting the antibiotic R106-I thus produced from the culture.

European Patent Office

# EUROPEAN SEARCH REPORT

Application Number

EP 92 12 0936

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 443 719 (TAKARA SHUZO CO. LTD.) * reference example * * page 4, line 60 - page 5, line 16; examples 1-7 * | 1 | C12P21/04 C07K11/02 //(C12P21/04, C12R1:645) |
| D,X | EP-A-0 352 092 (TAKARA SHUZO CO. LTD.) * compound 1 * * page 3, line 59 - page 4, line 3; claims; examples 1,2 * | 1 | |
| | | | **TECHNICAL FIELDS SEARCHED (Int. Cl.5 )** |
| | | | C07K |

The present search report has been drawn up for all claims

| Place of search | Date of completion of the search | Examiner |
|---|---|---|
| THE HAGUE | 23 FEBRUARY 1993 | FUHR C.K.B. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)